# EUROPEAN PATENT SPECIFICATION

(11) **EP 4 143 848 B1**
(45) Date of publication and mention of the grant of the patent: **03.04.2024**
(21) Application number: 21724356.7
(22) Date of filing: 23.04.2021
(51) Int. Cl.: G16H 50/20, G16H 50/70

(54) **PATIENT STRATIFICATION USING LATENT VARIABLES**
PATIENTENSTRATIFIZIERUNG MIT LATENTEN VARIABLEN
STRATIFICATION DES PATIENTS À L'AIDE DE VARIABLES LATENTES

(30) Priority: 28.04.2020 GB 202006264
(43) Date of publication of application: 08.03.2023
(73) Proprietor: BenevolentAI Technology Limited, London Greater London W1T 5HD (GB)
(72) Inventor: SIM, Aaron, London Greater London W1T 5HD (GB); CREED, Paidi, London Greater London W1T 5HD (GB); ZHANG, Jiajie, London Greater London W1T 5HD (GB); GLASTONBURY, Craig, London Greater London W1T 5HD (GB); NORVAISAS, Povilas, London Greater London W1T 5HD (GB); MULAS, Francesca, London Greater London W1T 5HD (GB); LEUG, Gregor Alexander, London Greater London W1T 5HD (GB); WATCHARAPICHAT, Pijika, London Greater London W1T 5HD (GB)
(74) Representative: Gill Jennings & Every LLP
(86) International application number: PCT/GB2021/050998
(87) International publication number: WO 2021/219980

(56) References cited:
- WO-A1-2018/140256
- US-A1- 2019 065 666

## Description

The present application relates to systems and methods for stratifying a population of patients into disease endotypes. The presently disclosed techniques find particular application in the fields of bioinformatics and drug discovery where there is a need to understand disease endotypes and develop treatments for them.

### Background

In order to stratify a population of patients into disease endotypes, a model is needed that can take data relating to the population of patients and use it to separate the patients into groups corresponding to disease endotypes. However, often not enough is known about the biological processes underlying the disease and its endotypes to successfully model them. As a result, when a disease is not well understood it can be difficult to stratify a population of patients into disease endotypes.

Accordingly, there is a need for an improved technique for identifying disease endotypes when the disease and its underlying biological mechanisms are not well understood.

The embodiments described below are not limited to implementations which solve any or all of the disadvantages of the known approaches described above.

US 2019/065666 A1 discloses methods and compositions generally relating to methods of identifying, validating, and measuring clinically relevant, quantifiable biomarkers of diagnostic and therapeutic responses for blood, vascular, cardiac, and respiratory tract dysfunction, particularly as those responses relate to septic shock in paediatric patients.

WO 2018/140256 A1 discloses methods for determining whether a subject has sepsis, or is at risk of developing sepsis, and methods of treating the subject based on the determination.

### Summary

This Summary is provided to introduce a selection of concepts in a simplified form that are further described below in the Detailed Description. This Summary is not intended to identify key features or essential features of the claimed subject matter, nor is it intended to be used to determine the scope of the claimed subject matter.

In a first aspect, the present disclosure provides a computer-implemented method of stratifying a population of patients into disease endotypes in accordance with appended claim 1.

Optionally, the data comprises one or more of genomics data, transcriptomics data, methylation data, copy number variation data, proteomics data, and clinical data. Optionally, the computer-implemented method comprises performing batch correction on the data. Optionally, the computer-implemented method comprises encoding the data using an unsupervised machine learning model. Optionally, the machine learning model comprises one or more from the group comprising a linear factor model, an autoencoder and a non-linear variational autoencoder. Optionally, the computer-implemented method comprises applying sparsity constraints to the latent variables. Optionally, the computer-implemented method comprises extracting from the data a copy of labelled data; and using the latent variables to predict clinical attributes. Optionally, the computer-implemented method comprises running one or more unsupervised machine learning models repeatedly to encode the data multiple times, wherein determining one or more importance measures of a latent variable comprises determining an extent of recurrence of the latent variable. Optionally, determining one or more importance measures of a latent variable comprises: determining a contribution of the latent variable to a proportion of variation. Optionally, determining one or more importance measures of a latent variable comprises: determining an ability of the latent variable to separate patients from a control group. Optionally, determining one or more importance measures of a latent variable comprises: determining an extent to which the latent variable is predictive of a patient attribute. Optionally, prioritising the latent variables using the importance measures comprises: rewarding a latent variable that is predictive of a patient attribute that is relevant to the disease. Optionally, the patient attribute that is relevant to the disease comprises one of: patient survival time, a quality of life measure, a disease stage and a likelihood of relapse. Optionally, prioritising the latent variables using the importance measures comprises: penalising a latent variable that is predictive of a patient attribute that is not relevant to the disease. Optionally, the patient attribute that is not relevant to the disease comprises one of: race and gender. Optionally, interpreting one or more of the latent variables comprises: applying gene enrichment analysis to the one or more latent variables. Optionally, identifying a disease endotype that is represented by one or more of the interpreted latent variables comprises: identifying a biological process underlying the disease using a gene expression pattern encoded in the one or more latent variables.

In a second aspect, the present disclosure provides a computer-readable medium storing code that, when executed by a computer, causes the computer to perform the above method.

In a third aspect, the present disclosure provides a system for stratifying a population of patients into disease endotypes in accordance with appended claim 12.

Optionally, the encoder comprises a batch correction module configured to perform batch correction on the data. Optionally, the encoder comprises an unsupervised machine learning module. Optionally, the unsupervised machine learning module comprises one or more from the group comprising a linear factor model, an autoencoder and a non-linear variational autoencoder. Optionally, the encoder comprises: an extraction tool configured to extract from the data a copy of labelled data; and a prediction module configured to use the latent variables to predict patient attributes. Optionally, the importance module comprises a comparison tool configured to: compare the labelled data and the predicted patient attributes; and determine an importance measure of a latent variable by determining an extent to which the latent variable is predictive of the patient attribute. Optionally, the interpretation module comprises a gene enrichment module configured to apply gene enrichment analysis to the one or more latent variables.

The methods described herein may be performed by software in machine readable form on a tangible storage medium e.g. in the form of a computer program comprising computer program code means adapted to perform all the steps of any of the methods described herein when the program is run on a computer and where the computer program may be embodied on a computer readable medium. Examples of tangible (or non-transitory) storage media include disks, thumb drives, memory cards etc. and do not include propagated signals. The software can be suitable for execution on a parallel processor or a serial processor such that the method steps may be carried out in any suitable order, or simultaneously.

This application acknowledges that firmware and software can be valuable, separately tradable commodities. It is intended to encompass software, which runs on or controls "dumb" or standard hardware, to carry out the desired functions. It is also intended to encompass software which "describes" or defines the configuration of hardware, such as HDL (hardware description language) software, as is used for designing silicon chips, or for configuring universal programmable chips, to carry out desired functions.

The preferred features may be combined as appropriate, as would be apparent to a skilled person, and may be combined with any of the aspects of the invention.

### Brief Description of the Drawings

Embodiments of the invention will be described, by way of example, with reference to the following drawings, in which:
Figure 1 is a block diagram of a system for stratifying a population of patients into disease endotypes according to an embodiment of the invention;
Figure 2 is a flow chart of a method that may be carried out by the above system according to an embodiment of the invention;
Figure 3 is a schematic diagram representing diagrammatically the steps of the above method;
Figure 4 is a block diagram of example input data that may be received by the above system of according to an embodiment of the invention;
Figure 5 is a block diagram of an example of the above system showing optional features;
Figure 6 is a flow chart showing an example of the above method showing optional steps;
Figure 7 is a schematic diagram of an autoencoder suitable for use in embodiments of the invention; and
Figure 8 is a block diagram of a computer hardware suitable for implementing embodiments of the invention.

Common reference numerals are used throughout the figures to indicate similar features.

### Detailed Description

Embodiments of the present invention are described below by way of example only. These examples represent the best ways of putting the invention into practice that are currently known to the Applicant although they are not the only ways in which this could be achieved. The description sets forth the functions of the example and the sequence of steps for constructing and operating the example. However, the same or equivalent functions and sequences may be accomplished by different examples.

When a patient population is successfully stratified into disease endotypes, this provides an opportunity to better understand the endotypes in order to discover the biological mechanisms underlying them. When the biological mechanisms of disease endotypes are understood, this opens up the possibilities for treatments to be developed.

However, when a disease and its endotypes are not well understood, it can be difficult to get started because often not enough is known about the disease to build a model that can separate patients into the endotypes of the disease.

One way of dealing with this is to use unsupervised machine learning techniques to model the disease. In this case, the model itself assigns latent variables to the disease and does not require prior knowledge of which variables are of interest. Latent variables are non-observable factors that can reveal disease endotypes from among a seemingly homogenous population of patients. As a result of this approach, the biological mechanisms underlying the disease and its endotypes are discoverable using the latent variables assigned by the model, and there is no need for a detailed, manual construction of complex models based on prior knowledge of the disease.

The present invention provides systems and methods for stratifying a population of patients using latent variables. Systems according to the invention receive as input data relating to a population of patients and deliver as output a stratification of patients.

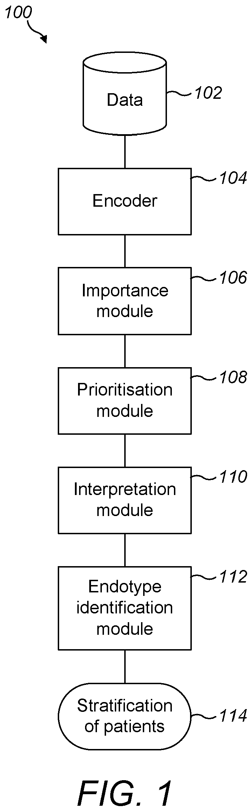

Figure 1 shows a system 100 for stratifying a population of patients into disease endotypes according to an embodiment of the invention. The system 100 receives data 102 relating to patients which it uses to stratify the patients into subgroups of the disease or 'disease endotypes', which are subtypes of the same disease that have different underlying biological mechanisms. Phenotypes are sets of observable traits useful for identifying subpopulations of similar patients. As a result, when phenotypes have been linked to a distinct underlying pathobiological mechanism, they may be referred to as disease endotypes.

In order to achieve this, the system 100 comprises an encoder 104 that is configured to encode the data 102 as latent variables. This type of encoding allows groupings of related biological features such as genes to be extracted as latent variables from the data without introducing unnecessary assumptions. Each latent variable represents a different grouping of related biological features that together may represent an underlying biological mechanism. As a result, encoding the data as latent variables provides a way of separating different mechanisms of the same disease into separate groups to stratify patients into endotypes.

The encoder 104 may typically extract hundreds or thousands of latent variables from the data 102, so it is necessary to assess which latent variables are potentially meaningful and worth investigating further, and which are more likely to be artefacts with no biological significance that can safely be ignored. For this purpose, the system 100 comprises an importance module 106 for determining the importance of each latent variable by evaluating a range of measures for each latent variable that provide an indication of the likelihood that the latent variable is meaningful and has a biological significance that is useful for investigating the disease under study. In this document these measures will be referred to as `importance measures' and fall into two main categories: technical importance measures and biological importance measures. Technical importance measures are statistical measures of the value of a latent variable that provide an indication of whether a latent variable is statistically meaningful rather than just being an artefact. For example, if a latent variable is recurring when the data 102 is encoded repeatedly, then it is likely to be statistically meaningful and unlikely to be an artefact. Biological importance measures are measures of whether a latent variable appears to have a biological significance that is useful for investigating the disease under study. For example, if a latent variable is predictive of whether a patient has a disease or belongs to a control group that does not have the disease, then the latent variable has a biological significance that may be useful for investigating the disease. If a latent variable is not predictive of whether or not a patient has the disease, then it may not have a biological significance that is useful for investigating the disease. In order for a latent variable to be considered to represent a disease endotype, it will need to satisfy a minimum standard of significance as indicated by the technical importance measures, and will also need to satisfy a minimum standard of significance as indicated by the biological importance measures. As a result, it can be said with confidence that a latent variable being interpreted as representing a disease endotype is biologically meaningful and is not a statistical artefact.

The importance module 106 outputs values of importance measures for each latent variable that can be used to determine the overall importance of each latent variable for the disease. The system 100 comprises a prioritisation module 108 that receives the importance measures and determines an overall priority for each latent variable using the importance measures. For example, this may be in the form of a ranking of the latent variables from most important to least important for the disease.

The system 100 comprises an interpretation module 110 configured to interpret one or more of the latent variables. Interpretation is be restricted to top priority latent variables, namely for those having a priority above a threshold that are considered to satisfy minimum requirements for being potentially useful for investigating the disease. Each latent variable that is considered worth investigating further is J interpreted by identifying genes that it encodes and applying gene enrichment analysis to identify any pathological gene expression patterns that it may represent. For example, a gene expression pattern represented by a latent variable can be tested to determine whether the genes that it encodes are significantly enriched for a given cell type, disease mechanism or biological process. As such, latent variables can be interpreted to identify underlying pathologies that they may represent.

Finally, the system 100 comprises an endotype identification module 112 configured to identify a disease endotype that is represented by one or more of the interpreted latent variables. For example, there may be a latent variable that represents an underlying pathology that is associated with symptoms of the disease. In this case, it may be determined that the latent variable represents an endotype of the disease. The endotype identification module 112 may then finally output a result comprising a stratification 114 of patients into disease endotypes.

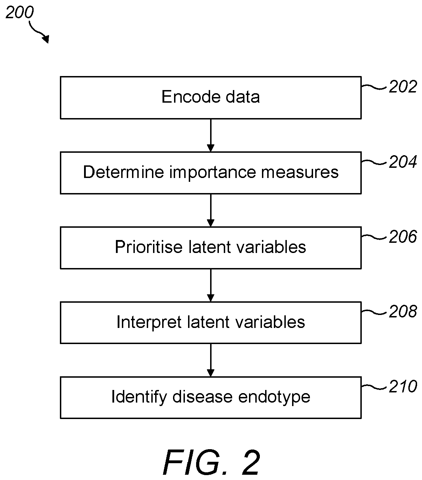

With reference to Figure 2, the present disclosure extends to a computer-implemented method 200 of stratifying a population of patients into disease endotypes. The method may be carried out by the system 100 of Figure 1 and comprises: encoding 202 data relating to the patients as latent variables; determining 204 one or more importance measures of the latent variables; proiritising 206 the latent variables using the importance measures; interpreting 208 one or more of the latent variables; and identifying 210 a disease endotype that is represented by one or more of the interpreted latent variables.

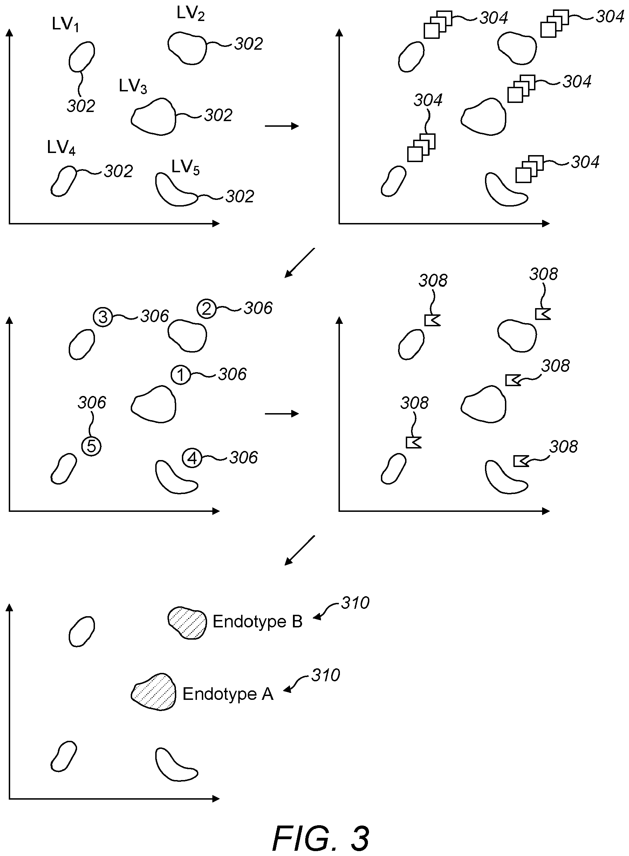

An example of the method 200 is represented diagrammatically in Figure 3. With reference to Figure 3, patient data is encoded 202 as latent variables 302 that form groupings of related biological features such as gene expression patterns and protein levels that can be used to stratify patients into endotypes.

One or more importance measures 304 are then determined 204 for each latent variable 302 that provide an indication of how important the latent variable is for investigating the disease. As described above, these importance measures may be technical importance measures that provide an indication of whether a latent variable is statistically meaningful rather than an artefact, or they may be biological importance measures that provide a measure of whether a latent variable appears to have a biological significance that is useful for investigating the disease. There may suitably be tens of importance measures determined for each latent variable.

On the basis of the importance measures 304, the latent variables 302 are then prioritised 206 and assigned a priority or rank 306 that reflects their overall importance for investigating the disease. For example, as shown in Figure 3, the latent variables 302 are each assigned a rank from the most important which has a rank of '1' to the least important which has a rank of '5'. When determining an overall priority or rank 306 for the latent variables 302, the different importance measures 304 may suitably be weighted according to which importance measures are considered to have a greater influence on the importance of a latent variable 302 for investigating the disease. For example, a biological importance measure 304 indicating that a latent variable 302 is highly predictive of survival time in cancer patients may be assigned a large weighting.

One or more of the latent variables 302 are interpreted 208 for biological meaning. As shown in Figure 3, interpretations 308 are assigned to the latent variables 302. In the example of Figure 3, all the latent variables 302 are assigned interpretations 308, but in other examples it may be suitable to interpret only a subset of the latent variables 302, for example if there are large numbers of latent variables 302 and low ranking latent variables 302 are considered to be artefacts or not relevant to the disease.

Finally, disease endotypes 310 represented by the latent variables 302 are identified 210. In the example of Figure 3, two disease endotypes 310 are identified. The other three latent variables 302 may be artefacts or may not be relevant to the disease or may not represent a disease endotype for other reasons.

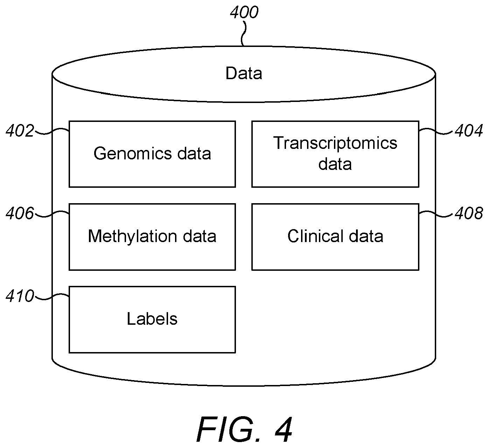

Referring to Figure 4, an exemplary set of data 400 provides an example of the data 102 that is received by the system 100 according to an embodiment of the invention. The data 102 relates to patients in a disease population and may be measured in multiple experiments such as micro arrays, genomics and RNA sequencing. A suitable data set 102 may, for example, be based on data from approximately 100 patients although this figure is non-limiting and not intended as a guide. Non-limiting examples of data 102 include gene expression data, presence of a gene or gene variant, genotyping data, methylation data, copy number variation data, proteomics data, and clinical data such as disease onset.

In the example of Figure 4, the exemplary data set 400 comprises genomics data 402, transcriptomics data 404, methylation data 406 and clinical data 408.

The data 102 may also include labels such as the labels 410 show in Figure 4. Labels provide additional information about the patient population and can be used to assess the value of latent variables as described further below. Non-limiting examples of labels include patient survival times, metadata from when the data was collected, patient data from questionnaires, data from electronic health records, data such as blood type, patient age, patient gender and clinical outcomes from longitudinal studies, data relating to mutations of specific DNA regions, biomarker measurements, and scores indicating disease progression.

Any missing data can be handled by adopting a probabilistic approach in which the model that encodes the data as latent variables treats missing data as unknown parameters that can be statistically inferred.

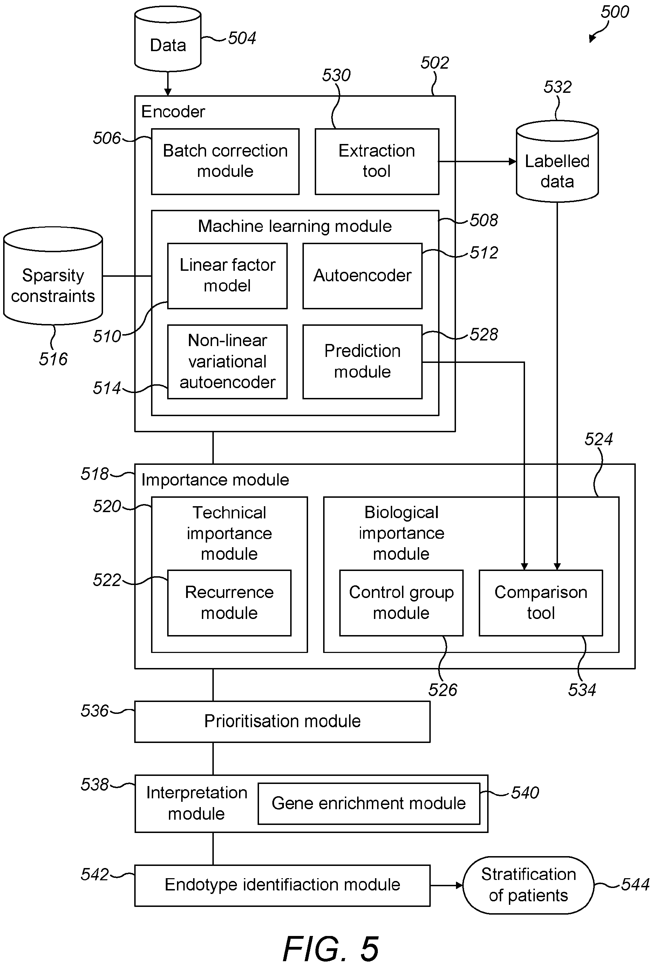

Figure 5 shows an example 500 of the above system 100. System 500 comprises an encoder 502 configured to receive data 504 relating to patients that could, for example, comprise the data set 400 shown in Figure 4. The encoder 502 is configured to encode the data 504 as latent variables.

The encoder 502 comprises a batch correction module 506 configured to perform batch correction on the data 504. The batch correction may improve at least one signal-to-noise ratio and may, for example, be performed using linear models to remove experimental effects that increase signal-to-noise ratios.

The encoder 502 comprises a machine learning module 508 that uses unsupervised machine learning techniques to encode the data 504 as latent variables. Since the machine learning is unsupervised, any labels 410 in the data 504 are not used as inputs for the encoding. In alternatives to the example 500 of Figure 5, the machine learning module 508 may include machine learning models that use some of the labels 410 as inputs, which would result in models with additional features.

The machine learning module 508 of Figure 5 comprises a linear factor model 510, an autoencoder 512 and a non-linear variational autoencoder 514. It will be appreciated that in other example systems the machine learning module 508 may comprise one or more from the group comprising a linear factor model 510, an autoencoder 512 and a non-linear variational autoencoder 514, either alone or in combination with any one or more other suitable unsupervised machine learning models.

The machine learning model 508 is configured to apply sparsity constraints 516 to the latent variables. Sparsity constraints 516 may be applied to loading matrices for linear factor models 510 and to encoder and decoder neural networks of the autoencoder 512 and of the non-linear variational autoencoder 514. The application of sparsity constraints 516 aids interpretation of the latent variables.

The system 500 comprises an importance module 518 configured to determine one or more importance measures of the latent variables.

The importance module 518 comprises a technical importance module 520 configured to determine technical importance measures of the latent variables. The technical importance module 520 comprises a recurrence module 522 configured to determine an extent of recurrence of a latent variable when one or more of the unsupervised machine learning models 510, 512, 514 are run multiple times. When a latent variable is recurring, this generally indicates that the model that repeatedly generates it is robust.

The technical importance module 520 may also be configured to determine a technical importance measure of a latent variable by determining a contribution of the latent variable to a proportion of variation.

Technical importance measures provide a measure of whether a latent variable is statistically meaningful rather than just being an artefact. By way of example, further non-limiting technical importance measures may relate to the following considerations.
- What is the sparsity pattern of a latent variable?
- Does a latent variable come up consistently across model runs?
- For each latent variable, what other latent variables are similar to it?

The importance module 518 also comprises a biological importance module 524 configured to determine biological importance measures of the latent variables. The biological importance module 524 comprises a control group module 526 configured to determine an ability of a latent variable to separate patients from a control group. In this case the patients belong to a disease population whereas the individuals in the control group do not.

Some importance measures of the latent variables have to do with whether the latent variables are predictive of patient attributes such as survival times and gender. For example, survival times are highly relevant for cancer patients, so a latent variable that is predictive of survival times will be useful for investigating the disease. In this case, a latent variable that is predictive of survival times will be assigned an importance measure that has the effect of prioritising or up-ranking the latent variable. By contrast, race is not typically relevant for cancer patients, so a latent variable that is predictive of race will be unlikely to be useful for investigating the disease. In fact, the correlation with race would suggest that the latent variable has a biological meaning that is not relevant to the disease. In this case, the latent variable will be assigned an importance measure that has the effect of deprioritising or down-ranking the latent variable.

In order to accommodate these types of importance measures, the system 500 is configured to assess the extent to which a latent variable is predictive of patient attributes such as survival time and race. Referring to Figure 5, the machine learning module 508 of the system 500 comprises a prediction module 528 configured to use the latent variables to predict patient attributes. The accuracy of the predictions generated by the prediction module 528 will depend on how well the latent variables are correlated with the patient attributes. Furthermore, the encoder 502 comprises an extraction tool 530 configured to extract from the data 504 a copy of labelled data 532 which can be fed into the importance module 518. The labelled data comprises patient data together with labels of known patient attributes that the prediction module 528 is trying to predict based on the learned latent variables. For example, if the data 504 relates to a population of cancer patients, then the data 504 would suitably include a subset labelled with survival times. In this case, the machine learning module 508 is configured to encode the data as latent variables based on the unlabelled data and latent variables are used to make predictions. In order to assess the accuracy of the predictions, the biological importance module 524 comprises a comparison tool 534 that receives predictions from the prediction module 528 and labelled data from the extraction tool 530. The comparison tool 534 is configured to compare the labelled data and the predicted patient attributes and to determine an importance measure of a latent variable by determining an extent to which the latent variable is predictive of the patient attribute. If the latent variable is predictive of a patient attribute that is relevant to the disease (e.g. survival time), the comparison tool 534 is likely to reward the latent variable by assigning an up-ranking importance measure to the latent variable. If the latent variable is predictive of a patient attribute that is not relevant to the disease (e.g. race), the comparison tool 534 is likely to penalise the latent variable by assigning a down-ranking importance measure to the latent variable. Other examples of patient attributes that may be relevant to a disease include quality of life measures, disease stage and likelihood of relapse.

Some further non-limiting examples of biological importance measures may relate to the following considerations.
- Do genes encoded in the latent variable enrich for known gene signatures?
- Does the latent variable distinguish known sample groups of patients?
- Does the latent variable correlate with patient survival?
- Do the expression levels of genes encoded in a latent variable correlate with a given continuous measurement such as patient age?
- Do patient subgroups defined by latent variables enrich for labelled phenotypes?
- Do patient subgroups defined by latent variables have a significant difference in their metadata values (such as age, gender, tumour stage, etc)
- What are the upstream regulators of genes encoded in a latent variable and do the upstream regulators include genes or proteins of interest?
- Are there druggable upstream regulators of genes encoded in a latent variable?
- Are genes encoded in a latent variable more connected in patients having the disease than a control group not having the disease?
- What are the genes encoded by latent variables that sufficiently differentiate between endotypes?

The system 500 comprises a prioritisation module 536 configured to receive the importance measures from the importance module 518 and to determine an overall priority or ranking for each latent variable based on the importance measures.

The system 500 comprises an interpretation module 538 configured to interpret some or all of the latent variables. The interpretation module 538 comprises a gene enrichment module 540 configured to apply gene enrichment analysis to the one or more latent variables. The gene enrichment module 540 may be configured to use standard gene ontology libraries and/or curated gene sets representing biological processes to interpret the latent variables. For interpreting linear latent variables generated by the linear factor model 510, the interpretation module 538 may be configured to extract interpretations from load matrices. For interpreting latent variables generated by the autoencoder 512 and/or the non-linear variational autoencoder 514, the interpretation module 538 may be configured to use a variety of attribution methods, including gene enrichment, to associate features such as genes with each latent variable. These methods enable the interpretation module to identify a biological meaning such as a biological mechanism which could be an underlying mechanism of a disease endotype to be associated with a latent variable.

Finally, the system 500 comprises an endotype identification module 542. The endotype identification module 542 is configured to identify a biological process underlying the disease using a gene expression pattern encoded in the one or more latent variables. The endotype identification module 542 is thereby configured to associate a disease endotype having an underlying biological process with one or more latent variables and to output a result comprising a stratification 544 of patients into disease endotypes.

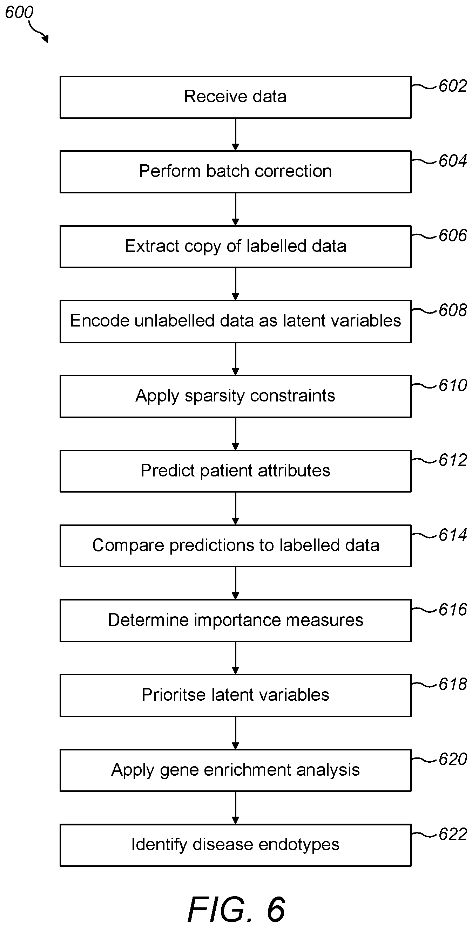

With reference to Figure 6, the present disclosure extends to a computer-implemented method 600 of stratifying a population of patients into disease endotypes. The method 600 is an example of the above method 200 and may be carried out by the system 500 of Figure 5. The method 600 comprises receiving 602 data relating to patients and performing 604 batch correction on the data, for example to improve at least one signal-to-noise ratio. The method 600 comprises extracting 606 a copy of labelled data from the received data and encoding 608 unlabelled data from the data as latent variables. To aid interpretation of the latent variables, the method 600 comprises applying 610 sparsity constraints to the latent variables.

In order to assess the extent to which latent variables are predictive of patient attributes such as survival time and race, the method 600 comprises predicting 612 patient attributes using the latent variables and comparing 614 the predictions to the labelled data. The method 600 comprises determining 616 importance measures for the latent variables which may comprise determining the extent to which a latent variable is predictive of a patient attribute. For example, a latent variable that is predictive of a patient attribute that is relevant to the disease under study (e.g. survival time for cancers) may be assigned an importance measure that has the effect of prioritising or up-ranking the latent variable. In another example, a latent variable that is predictive of a patient attribute that is not relevant to the disease under study (e.g. race for many diseases) may be assigned an importance measure that has the effect of deprioritising or down-ranking the latent variable.

The method 600 comprises prioritising the latent variables 618 on the basis of the importance measures. Some importance measures may be given a greater weighting in the aggregated, overall priority assigned to the latent variables. For example, an important measure related to survival times for cancers may be given a large weighting because survival time is highly relevant for cancers. The step of prioritising 618 the latent variables results in a prioritised set of latent variables which may for example take the form of a ranking.

The method 600 comprises applying 620 gene enrichment analysis to the latent variables to aid their interpretation. Finally, the method 600 comprises identifying 622 disease endotypes associated with the latent variables.

It will be appreciated that the steps of method 600 do not necessarily have to be performed in the order described above and shown in Figure 6. For example, the unlabelled data may be encoded 608 before a copy of the labelled data is extracted 606.

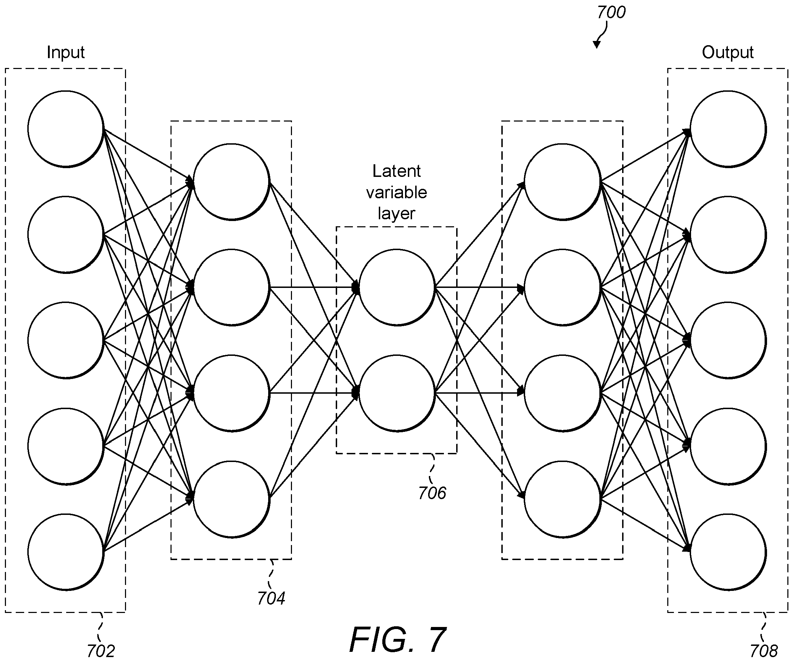

Figure 7 shows an example of an autoencoder 700 with which latent variables may be generated. In this example, an input vector 702 is passed through a neural network of one or more layers of hidden nodes 704 to an intermediate layer with fewer nodes than the input - that is with a dimensionality reduction 706. These nodes are connected to additional nodes in additional layers to a series of output nodes 708 of the same dimensionality as the input layer. Such a system may be trained to reconstruct input data at the output, resulting in compact, lower dimensional representations of different inputs in the intermediate latent variable layer 706.

As an alternative, a variational autoencoder may be used that additionally encodes a standard deviation vector, which is sampled at the latent variable stage before being decoded back to the original input.

Additionally or alternatively, a further method for generating latent variable representations may be using unsupervised machine learning techniques or other clustering algorithms, such as k-means, mixture models, density-based spatial clustering of applications with noise (DBSCAN), or other methods. These methods may be linear or non-linear. It will be appreciated that latent variables may be generated using one of the above methods or a combination of those methods.

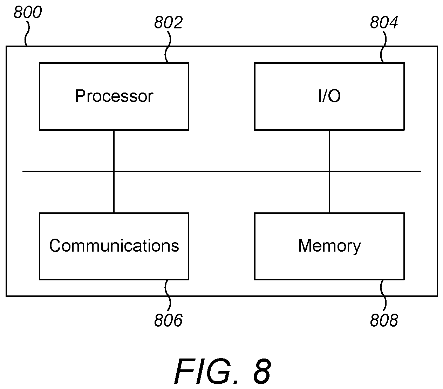

A computer apparatus 800 suitable for implementing methods according to the present invention is shown in Figure 8. The apparatus 800 comprises a processor 802, an input-output device 804, a communications portal 806 and computer memory 808. The memory 808 may store code that, when executed by the processor 802, causes the apparatus 800 to perform the method 200 shown in Figure 2.

In the embodiment described above the server may comprise a single server or network of servers. In some examples the functionality of the server may be provided by a network of servers distributed across a geographical area, such as a worldwide distributed network of servers, and a user may be connected to an appropriate one of the network of servers based upon a user location.

The above description discusses embodiments of the invention with reference to a single user for clarity. It will be understood that in practice the system may be shared by a plurality of users, and possibly by a very large number of users simultaneously.

The embodiments described above are fully automatic. In some examples a user or operator of the system may manually instruct some steps of the method to be carried out.

In the described embodiments of the invention the system may be implemented as any form of a computing and/or electronic device. Such a device may comprise one or more processors which may be microprocessors, controllers or any other suitable type of processors for processing computer executable instructions to control the operation of the device in order to gather and record routing information. In some examples, for example where a system on a chip architecture is used, the processors may include one or more fixed function blocks (also referred to as accelerators) which implement a part of the method in hardware (rather than software or firmware). Platform software comprising an operating system or any other suitable platform software may be provided at the computing-based device to enable application software to be executed on the device.

Various functions described herein can be implemented in hardware, software, or any combination thereof. If implemented in software, the functions can be stored on or transmitted over as one or more instructions or code on a computer-readable medium. Computer-readable media may include, for example, computer-readable storage media. Computer-readable storage media may include volatile or non-volatile, removable or non-removable media implemented in any method or technology for storage of information such as computer readable instructions, data structures, program modules or other data. A computer-readable storage media can be any available storage media that may be accessed by a computer. By way of example, and not limitation, such computer-readable storage media may comprise RAM, ROM, EEPROM, flash memory or other memory devices, CD-ROM or other optical disc storage, magnetic disc storage or other magnetic storage devices, or any other medium that can be used to carry or store desired program code in the form of instructions or data structures and that can be accessed by a computer. Disc and disk, as used herein, include compact disc (CD), laser disc, optical disc, digital versatile disc (DVD), floppy disk, and blu-ray disc (BD). Further, a propagated signal is not included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable, twisted pair, DSL, or wireless technologies such as infrared, radio, and microwave are included in the definition of communication medium. Combinations of the above should also be included within the scope of computer-readable media.

Alternatively, or in addition, the functionality described herein can be performed, at least in part, by one or more hardware logic components. For example, and without limitation, hardware logic components that can be used may include Field-Programmable Gate Arrays (FPGAs), Program-Specific Integrated Circuits (ASICs), Program-Specific Standard Products (ASSPs), System-On-a-Chip systems (SOCs). Complex Programmable Logic Devices (CPLDs), etc.

Although illustrated as a single system, it is to be understood that the computing device may be a distributed system. Thus, for instance, several devices may be in communication by way of a network connection and may collectively perform tasks described as being performed by the computing device.

Although illustrated as a local device it will be appreciated that the computing device may be located remotely and accessed via a network or other communication link (for example using a communication interface).

The term 'computer' is used herein to refer to any device with processing capability such that it can execute instructions. Those skilled in the art will realise that such processing capabilities are incorporated into many different devices and therefore the term 'computer' includes PCs, servers, mobile telephones, personal digital assistants and many other devices.

Those skilled in the art will realise that storage devices utilised to store program instructions can be distributed across a network. For example, a remote computer may store an example of the process described as software. A local or terminal computer may access the remote computer and download a part or all of the software to run the program. Alternatively, the local computer may download pieces of the software as needed, or execute some software instructions at the local terminal and some at the remote computer (or computer network). Those skilled in the art will also realise that by utilising conventional techniques known to those skilled in the art that all, or a portion of the software instructions may be carried out by a dedicated circuit, such as a DSP, programmable logic array, or the like.

Any reference to "an" item refers to one or more of those items. The term 'comprising' is used herein to mean including the method steps or elements identified, but that such steps or elements do not comprise an exclusive list and a method or apparatus may contain additional steps or elements.

As used herein, the terms "component" and "system" are intended to encompass computer-readable data storage that is configured with computer-executable instructions that cause certain functionality to be performed when executed by a processor. The computer-executable instructions may include a routine, a function, or the like. It is also to be understood that a component or system may be localized on a single device or distributed across several devices.

Further, as used herein, the term "exemplary" is intended to mean "serving as an illustration or example of something".

Further, to the extent that the term "includes" is used in either the detailed description or the claims, such term is intended to be inclusive in a manner similar to the term "comprising" as "comprising" is interpreted when employed as a transitional word in a claim.

The figures illustrate exemplary methods. While the methods are shown and described as being a series of acts that are performed in a particular sequence, it is to be understood and appreciated that the methods are not limited by the order of the sequence. For example, some acts can occur in a different order than what is described herein. In addition, an act can occur concurrently with another act. Further, in some instances, not all acts may be required to implement a method described herein.

Moreover, the acts described herein may comprise computer-executable instructions that can be implemented by one or more processors and/or stored on a computer-readable medium or media. The computer-executable instructions can include routines, sub-routines, programs, threads of execution, and/or the like. Still further, results of acts of the methods can be stored in a computer-readable medium, displayed on a display device, and/or the like.

The order of the steps of the methods described herein is exemplary, but the steps may be carried out in any suitable order, or simultaneously where appropriate. Additionally, steps may be added or substituted in, or individual steps may be deleted from any of the methods without departing from the scope of the subject matter described herein. Aspects of any of the examples described above may be combined with aspects of any of the other examples described to form further examples without losing the effect sought.

## Claims

1. A computer-implemented method (200, 600) of stratifying a population of patients into disease endotypes, the method comprising:
encoding (202), using an autoencoder (700) trained to reconstruct input data (702) at an output (708), data (102, 400) relating to the patients as latent variables, wherein the latent variables comprise lower dimensional representations of the data relating to the patients in an intermediate layer of the autoencoder (706), the latent variables representing different groupings of related biological features;
determining (204) one or more importance measures of the latent variables, wherein the determining comprises encoding, using the autoencoder, the data relating to the patients a plurality of times and determining an extent of recurrence of the latent variables;
prioritising (206) the latent variables using the importance measures, the prioritising comprising ranking the latent variables from most important to least important for the disease;
interpreting (208) one or more of the latent variables above a priority threshold, wherein the interpreting comprises identifying genes that the latent variable encodes and identifying pathological gene expression patterns that the latent variables represent by applying gene enrichment analysis (620); and
identifying (210) a disease endotype that is represented by one or more of the interpreted latent variables, wherein the identifying comprises identifying a biological process underlying the disease using a gene expression pattern encoded in the one or more latent variables.

2. The computer-implemented method of claim 1, wherein the data comprises one or more of genomics data (402), transcriptomics data (404), methylation data (406), copy number variation data, proteomics data, and clinical data (408).

3. The computer-implemented method of claim 1 or 2, comprising performing (604) batch correction on the data.

4. The computer-implemented method of any preceding claim, comprising applying (610) sparsity constraints to the latent variables.

5. The computer-implemented method of any preceding claim, comprising:
extracting (606) from the data a copy of labelled data; and
using the latent variables to predict clinical attributes.

6. The computer-implemented method of any preceding claim, wherein determining one or more importance measures of a latent variable comprises:
determining a contribution of the latent variable to a proportion of variation.

7. The computer-implemented method of any preceding claim, wherein determining one or more importance measures of a latent variable comprises:
determining an ability of the latent variable to separate patients from a control group.

8. The computer-implemented method of any preceding claim, wherein determining one or more importance measures of a latent variable comprises:
determining an extent to which the latent variable is predictive of a patient attribute.

9. The computer-implemented method of claim 8, wherein prioritising the latent variables using the importance measures comprises:
rewarding a latent variable that is predictive of a patient attribute that is relevant to the disease, preferably wherein the patient attribute that is relevant to the disease comprises one of: patient survival time, a quality of life measure, a disease stage and a likelihood of relapse.

10. The computer-implemented method of claim 8 or 9, wherein prioritising the latent variables using the importance measures comprises:
penalising a latent variable that is predictive of a patient attribute that is not relevant to the disease, preferably wherein the patient attribute that is not relevant to the disease comprises one of: race and gender.

11. A computer-readable medium storing code that, when executed by a computer (800), causes the computer to perform the method of any previous claim.

12. A system (100, 500) for stratifying a population of patients into disease endotypes, the system comprising:
an encoder (104) configured to encode data relating to the patients as latent variables, the encoder comprising an autoencoder (700) trained to reconstruct input data (702) at an output (708), wherein the latent variables comprise lower dimensional representations of the data relating to the patients in an intermediate layer of the autoencoder (706), the latent variables representing different groupings of related biological features;
an importance module (106) configured to determine one or more importance measures of the latent variables, wherein the determining comprises encoding, using the autoencoder, the data relating to the patients a plurality of times, and wherein the importance module comprises a recurrence module (522) configured to determine an extent of recurrence of the latent variables;
a prioritisation module (108) configured to prioritise the latent variables using the importance measures, the prioritising comprising ranking the latent variables from most important to least important for the disease;
an interpretation module (110) configured to interpret one or more of the latent variables above a priority threshold, wherein the interpreting comprises identifying genes that the latent variable encodes, and wherein the interpretation module comprises a gene enrichment module (540) configured to identify pathological gene expression patterns that the latent variables represent by applying gene enrichment analysis (620); and
an endotype identification module (112) configured to identify a disease endotype that is represented by one or more of the interpreted latent variables, wherein the identifying comprises identifying a biological process underlying the disease using a gene expression pattern encoded in the one or more latent variables.

13. The system of claim 12, wherein the encoder comprises a batch correction module configured to perform batch correction on the data.

14. The system of claim 12 or 13, wherein the encoder comprises:
an extraction tool configured to extract from the data a copy of labelled data; and
a prediction module configured to use the latent variables to predict patient attributes.

15. The system of claim 14, wherein the importance module comprises a comparison tool configured to:
compare the labelled data and the predicted patient attributes; and
determine an importance measure of a latent variable by determining an extent to which the latent variable is predictive of the patient attribute.

## Patentansprüche

1. Computerimplementiertes Verfahren (200, 600) zur Stratifizierung einer Population von Patienten in Krankheitsendotypen, wobei das Verfahren Folgendes umfasst:
Kodieren (202), unter Verwendung eines Autoencoders (700), der zur Rekonstruktion von Eingabedaten (702) an einer Ausgabe (708) trainiert ist, von Daten (102, 400), die sich auf die Patienten beziehen, als latente Variablen, wobei die latenten Variablen niederdimensionale Repräsentationen der Daten, die sich auf die Patienten beziehen, in einer Zwischenschicht des Autoencoders (706) umfassen, wobei die latenten Variablen verschiedene Gruppierungen von verbundenen biologischen Merkmalen repräsentieren;
Bestimmen (204) von einem oder mehreren Wichtigkeitsmaßen der latenten Variablen, wobei das Bestimmen das Kodieren der Daten, die sich auf die Patienten beziehen, unter Verwendung eines Autoencoders viele Male und das Bestimmen eines Ausmaßes des Wiederauftretens der latenten Variablen umfasst;
Priorisieren (206) der latenten Variablen unter Verwendung der Wichtigkeitsmaße, wobei das Priorisieren das Einstufen der latenten Variablen von am wichtigsten bis am wenigsten wichtig für die Krankheit umfasst;
Interpretieren (208) von einer oder mehreren der latenten Variablen über einer Prioritätsschwelle, wobei das Interpretieren das Identifizieren von Genen, die die latente Variable kodiert, und das Identifizieren von pathologischen Genexpressionsmustern, die die latenten Variablen repräsentieren, durch Anwendung von Genanreicherungsanalyse (620) umfasst; und
Identifizieren (210) eines Krankheitsendotyps, der durch eine oder mehrere der interpretierten latenten Variablen repräsentiert wird, wobei das Identifizieren das Identifizieren eines biologischen Prozesses, der der Krankheit zugrunde liegt, unter Verwendung eines Genexpressionsmusters, das in der einen oder den mehreren latenten Variablen kodiert ist, umfasst.

2. Computerimplementiertes Verfahren nach Anspruch 1, wobei die Daten eine oder mehrere Arten von genomischen Daten (402), transkriptomische Daten (404), Methylierungsdaten (406), Kopienzahlvariationsdaten, proteomischen Daten und klinischen Daten (408) umfassen.

3. Computerimplementiertes Verfahren nach Anspruch 1 oder 2, das das Durchführen (604) von Batch-Korrektur mit den Daten umfasst.

4. Computerimplementiertes Verfahren nach einem vorstehenden Anspruch, das das Anwenden (610) von Sparsity-Einschränkungen auf die latenten Variablen umfasst.

5. Computerimplementiertes Verfahren nach einem vorstehenden Anspruch, das Folgendes umfasst:
Extrahieren (606) einer Kopie von gelabelten Daten aus den Daten; und
Verwenden der latenten Variablen zur Vorhersage von klinischen Attributen.

6. Computerimplementiertes Verfahren nach einem vorstehenden Anspruch, wobei das Bestimmen von einer oder mehreren Wichtigkeitsmaßen einer latenten Variable Folgendes umfasst:
Bestimmen eines Beitrags der latenten Variable zu einem Anteil der Variation.

7. Computerimplementiertes Verfahren nach einem vorstehenden Anspruch, wobei das Bestimmen von einer oder mehreren Wichtigkeitsmaßen einer latenten Variable Folgendes umfasst:
Bestimmen einer Fähigkeit der latenten Variable zur Trennung von Patienten aus einer Kontrollgruppe.

8. Computerimplementiertes Verfahren nach einem vorstehenden Anspruch, wobei das Bestimmen von einer oder mehreren Wichtigkeitsmaßen einer latenten Variable Folgendes umfasst:
Bestimmen eines Ausmaßes, in wieweit die latente Variable für ein Patientenattribut vorhersagend ist.

9. Computerimplementiertes Verfahren nach Anspruch 8, wobei das Priorisieren der latenten Variablen unter Verwendung der Wichtigkeitsmaße Folgendes umfasst:
Belohnen einer latenten Variable, die für ein Patientenattribut vorhersagend ist, das für die Krankheit relevant ist, bevorzugt wobei das Patientenattribut, das für die Krankheit relevant ist, eines von Überlebenszeit des Patienten, einem Maß der Lebensqualität, einem Krankheitsstadium und einer Wahrscheinlichkeit eines Rückfalls umfasst.

10. Computerimplementiertes Verfahren nach Anspruch 8 oder 9, wobei das Priorisieren der latenten Variablen unter Verwendung der Wichtigkeitsmaße Folgendes umfasst:
Bestrafen einer latenten Variable, die für ein Patientenattribut vorhersagend ist, das nicht für die Krankheit relevant ist, bevorzugt wobei das Patientenattribut, das nicht für die Krankheit relevant ist, eines von Abstammung und Geschlecht umfasst.

11. Computerlesbarer Medienspeichercode, der bei Ausführung durch einen Computer (800) bewirkt, dass der Computer das Verfahren nach einem vorstehenden Anspruch ausführt.

12. System (100, 500) zur Stratifizierung einer Population von Patienten in Krankheitsendotypen, wobei das System Folgendes umfasst:
einen Kodierer (104), der zur Kodierung von Daten, die sich auf die Patienten beziehen, als latente Variablen konfiguriert ist, wobei der Kodierer einen Autoencoder (700) umfasst, der zur Rekonstruktion von Eingabedaten (702) an einer Ausgabe (708) trainiert ist, wobei die latenten Variablen niederdimensionale Repräsentationen der Daten, die sich auf die Patienten beziehen, in einer Zwischenschicht des Autoencoders (706) umfassen, wobei die latenten Variablen verschiedene Gruppierungen von verbundenen biologischen Merkmalen repräsentieren;
ein Wichtigkeitsmodul (106), das zur Bestimmung von einem oder mehreren Wichtigkeitsmaßen der latenten Variablen konfiguriert ist, wobei das Bestimmen das Kodieren der Daten, die sich auf die Patienten beziehen, unter Verwendung eines Autoencoders viele Male umfasst und wobei das Wichtigkeitsmodul ein Wiederauftrittsmodul (522) umfasst, das zur Bestimmung eines Ausmaßes des Wiederauftretens der latenten Variablen konfiguriert ist;
ein Priorisierungsmodul (108), das zur Priorisierung der latenten Variablen unter Verwendung der Wichtigkeitsmaße konfiguriert ist, wobei das Priorisieren das Einstufen der latenten Variablen von am wichtigsten bis am wenigsten wichtig für die Krankheit umfasst;
ein Interpretationsmodul (110), das zur Interpretierung von einer oder mehreren der latenten Variablen über einer Prioritätsschwelle konfiguriert ist, wobei das Interpretieren das Identifizieren von Genen, die die latente Variable kodiert, umfasst und wobei das Interpretationsmodul ein Genanreicherungsmodul (540) umfasst, das zur Identifizierung von pathologischen Genexpressionsmustern, die die latenten Variablen repräsentieren, durch Anwendung von Genanreicherungsanalyse (620) konfiguriert ist; und
ein Endotyp-Identifikationsmodul (112), das zur Identifizierung eines Krankheitsendotyps konfiguriert ist, der durch eine oder mehrere der interpretierten latenten Variablen repräsentiert wird, wobei das Identifizieren das Identifizieren eines biologischen Prozesses, der der Krankheit zugrunde liegt, unter Verwendung eines Genexpressionsmusters, das in der einen oder den mehreren latenten Variablen kodiert ist, umfasst.

13. System nach Anspruch 12, wobei der Kodierer ein Batch-Korrekturmodul umfasst, das zur Durchführung von Batch-Korrektur mit den Daten konfiguriert ist.

14. System nach Anspruch 12 oder 13, wobei der Kodierer Folgendes umfasst:
ein Extraktions-Tool, das zur Extraktion einer Kopie von gelabelten Daten aus den Daten konfiguriert ist; und
ein Vorhersagemodul, das zur Verwendung der latenten Variablen konfiguriert ist, um Patientenattribute vorherzusagen.

15. System nach Anspruch 14, wobei das Wichtigkeitsmodul ein Vergleichs-Tool umfasst, das für Folgendes konfiguriert ist:
Vergleichen der gelabelten Daten und der vorhergesagten Patientenattribute; und
Bestimmen eines Wichtigkeitsmaßes einer latenten Variable durch Bestimmen eines Ausmaßes, in wieweit die latente Variable für ein Patientenattribut vorhersagend ist.

## Revendications

1. Procédé mis en oeuvre par ordinateur (200. 600) de stratification d'une population de patients en des endotypes de maladie, le procédé comprenant :
encoder (202), en utilisant un auto-encodeur (700) formé pour reconstruire des données d'entrée (702) à une sortie (708), les données (102, 400) se rapportant à des patients comme des variables latentes, dans lequel les variables latentes comprennent des représentations dimensionnelles inférieures des données se rapportant aux patients dans une couche intermédiaire de l'auto-encodeur (706), les variables latentes représentant différents groupements de caractéristiques biologiques associées ;
déterminer (204) une ou plusieurs mesures d'importance des variables latentes, dans lequel déterminer comprend encoder, en utilisant l'auto-encodeur, les données se rapportant aux patients une pluralité de fois et déterminer un degré de récurrence des variables latentes ;
donner un ordre de priorité (206) aux variables latentes en utilisant des mesures d'importance, donner un ordre de priorité comprend classer les variables latentes de la plus importante à la moins importante pour la maladie ;
interpréter (208) une ou plusieurs variables latentes au-dessus d'un seuil de priorité, dans lequel interpréter comprend identifier des gènes que la variable latente encode et identifier des modèles d'expression de gènes pathologiques que les variables latentes représentent en appliquant une analyse d'enrichissement génique (620) ; et
identifier (210) un endotype de maladie qui est représenté par une ou plusieurs des variables latentes interprétées, dans lequel identifier comprend identifier un processus biologique sous-jacent à la maladie en utilisant un modèle d'expression génique encodé dans l'une ou plusieurs variables latentes.

2. Procédé mis en oeuvre par ordinateur selon la revendication 1, dans lequel les données comprennent une ou plusieurs d'entre des données génomiques (402), des données transcriptomiques (404), des données de méthylation (406), des données de variation dans le nombre de copies, des données protéomiques et des données cliniques (408).

3. Procédé mis en oeuvre par ordinateur selon la revendication 1 ou 2, comprenant effectuer (604) une correction par lots sur les données.

4. Procédé mis en oeuvre par ordinateur selon l'une quelconque des revendications précédentes, comprenant appliquer (610) des contraintes de parcimonie aux variables latentes.

5. Procédé mis en oeuvre par ordinateur selon l'une quelconque des revendications précédentes, comprenant :
extraire (606) des données une copie de données étiquetées ; et
utiliser les variables latentes pour prédire des attributs cliniques.

6. Procédé mis en oeuvre par ordinateur selon l'une quelconque des revendications précédentes, dans lequel déterminer une ou plusieurs mesures d'importance d'une variable latente comprend :
déterminer une contribution de la variable latente à une proportion de variation.

7. Procédé mis en oeuvre par ordinateur selon l'une quelconque des revendications précédentes, dans lequel déterminer une ou plusieurs mesures d'importance d'une variable latente comprend :
déterminer une aptitude de la variable latente à séparer des patients d'un groupe témoin.

8. Procédé mis en oeuvre par ordinateur selon l'une quelconque des revendications précédentes, dans lequel déterminer une ou plusieurs mesures d'importance d'une variable latente comprend :
déterminer un degré auquel la variable latente est prédictive d'un attribut de patient.

9. Procédé mis en oeuvre par ordinateur selon la revendication 8, dans lequel donner un ordre de priorité aux variables latentes en utilisant les mesures d'importance comprend :
gratifier une variable latente qui est prédictive d'un attribut de patient qui est pertinent à la maladie, de préférence dans lequel l'attribut de patient qui est pertinent à la maladie comprend l'un d'entre : durée de survie de patient, une qualité de mesure de vie, un stade de maladie et une probabilité de rechute.

10. Procédé mis en oeuvre par ordinateur selon la revendication 8 ou 9, dans lequel donner un ordre de priorité aux variables latentes en utilisant les mesures d'importance comprend :
pénaliser une variable latente qui est prédictive d'un attribut de patient qui n'est pas pertinent à la maladie, de préférence dans lequel l'attribut de patient qui n'est pas pertinent à la maladie comprend l'un d'entre : race et sexe.

11. Support de stockage lisible par ordinateur stockant du code qui, lorsque exécuté par un ordinateur (800) fait que l'ordinateur effectue le procédé selon l'une quelconque des revendications précédentes.

12. Système (100, 500) de stratification d'une population de patients en des endotypes de maladie, le système comprenant :
un encodeur (104) configuré pour encoder des données se rapportant à des patients comme des variables latentes, l'encodeur comprenant un auto-encodeur (700) formé pour reconstruire des données d'entrée (702) à une sortie (708), dans lequel les variables latentes comprennent des représentations dimensionnelles inférieures des données se rapportant aux patients dans une couche intermédiaire de l'auto-encodeur (706), les variables latentes représentant différents groupements de caractéristiques biologiques associées ;
un module d'importance (106) configuré pour déterminer une ou plusieurs mesures d'importance des variables latentes, dans lequel déterminer comprend encoder, en utilisant l'auto-encodeur, les données se rapportant aux patients une pluralité de fois, et dans lequel le module d'importance comprend un module de récurrence (522) configuré pour déterminer un degré de récurrence des variables latentes ;
un module de priorité (108) configuré pour donner un ordre de priorité aux variables latentes en utilisant les mesures d'importance, donner un ordre de priorité comprend classer les variables latentes de la plus importante à la moins importante pour la maladie ;
un module d'interprétation (110) configuré pour interpréter une ou plusieurs variables latentes au-dessus d'un seuil de priorité, dans lequel interpréter comprend identifier des gènes que la variable latente encode, et dans lequel le module d'interprétation comprend un module d'enrichissement génique (540) configuré pour identifier des modèles d'expression de gènes pathologiques que les variables latentes représentent en appliquant une analyse d'enrichissement génique (620) ; et
un module d'identification d'endotype (112) configuré pour identifier un endotype de maladie qui est représenté par une ou plusieurs des variables latentes interprétées, dans lequel identifier comprend identifier un processus biologique sous-jacent à la maladie en utilisant le modèle d'expression génique encodé dans une ou plusieurs variables latentes.

13. Système selon la revendication 12, dans lequel l'encodeur comprend un module de correction par lots configuré pour effectuer une correction par lots sur les données.

14. Système selon la revendication 12 ou 13, dans lequel l'encodeur comprend :
un outil d'extraction configuré pour extraire des données une copie de données étiquetées ; et
un module de prédiction configuré pour utiliser les variables latentes pour prédire des attributs de patient.

15. Système selon la revendication 14, dans lequel le module d'importance comprend un outil de comparaison configuré pour :
comparer les données étiquetées aux attributs prédits de patient ; et
déterminer une mesure d'importance d'une variable latente en déterminant un degré auquel la variable latente est prédictive de l'attribut de patient.
